# EUROPEAN PATENT APPLICATION

(11) **EP 4 725 414 A1**
(43) Date of publication of application: **15.04.2026**
(21) Application number: 24205768.5
(22) Date of filing: 10.10.2024
(51) Int. Cl.: A61B 5/1477, A61B 5/145, A61B 5/00, A61F 13/00

(54) **A SENSOR STRIP FOR USE IN CONJUNCTION WITH A MEDICAL DRESSING**

(71) Applicant: Mölnlycke Health Care AB, 431 21 Mölndal (SE)
(72) Inventor: WIRTHS, Walter, 82024 TAUFKIRCHEN (DE)
(74) Representative: Kransell & Wennborg KB

(57) **Abstract**

The present disclosure generally relates to a sensor strip (1) for use in conjunction with a medical dressing (2), wherein the sensor strip (1) comprises at least one sensing electrode (3) configured to measure the concentration of dissolved molecules in a wound, and at least a first (4a) and a second reference electrode (4b), characterized in that the at least first (4a) and second (4b) reference electrodes are individually distinct and differ by at least one property selected from set-up time, long-term-stability, life-time, and stability in response to chemical changes.

The present disclosure also relates to a medical dressing assembly (5) comprising the adhesive sensor strip (1) and to a wound monitoring system comprising the medical dressing assembly (5).

## Description

### TECHNICAL FIELD

The present disclosure generally relates to sensor strip comprising at least one sensing electrode for measuring the concentration of dissolved molecules in a wound, and at least a first and a second reference electrode. The present disclosure further relates to a medical dressing assembly comprising the sensor strip and to a wound monitoring system comprising the medical dressing assembly.

### BACKGROUND

Wound healing is a complicated process in which destroyed or damaged tissue is replaced with newly produced tissue.

The wound healing process is typically divided into four separate phases; i.e. hemostasis (blood clotting), inflammation, cell proliferation (tissue growth), and tissue remodeling (maturation and cell differentiation). For a wound to heal successfully, all these four phases must be accomplished. The wound healing process is susceptible to failure, which may lead to the formation of non-healing chronic wounds.

To evaluate and secure the progress of wound healing, wound assessment is critical. Clinicians typically assess various wound properties to secure that the wound healing process is proceeding in a proper manner. Factors that are typically assessed by the clinicians include the type of tissue, the level of exudate, as well as the color and characteristics of the exudate.

Traditionally, wound assessments have been largely qualitative, relying on visual inspections and subjective evaluations. Recent advancements have introduced the use of electrochemical sensors, including ion-selective electrodes, for quantitative wound analysis. These sensors can measure various parameters, e.g. pH level, which is indicative of wound status and healing progression.

Ion-selective electrodes operate based on the measurement of electrical potential differences between a working electrode sensitive to specific ions (e.g., H+ ions for pH measurement) and a non-sensitive reference electrode. The accuracy of these measurements is typically dependent on the stability and performance of the reference electrode.

Miniaturization and integration of sensors comprising sensing electrodes, such as ion-selective electrodes into wound dressings pose significant challenges, particularly with respect to the reliability and stability of the reference electrode.

It is generally challenging to achieve a balance between rapid sensor set-up, long-term stability, and accurate and reliable measurements. This is furthermore aggravated by the complexity of a wound environment, where factors such as changing exudate composition, pH variations, and biofilm formation can affect sensor performance.

There is consequently a need to improve and facilitate the wound assessment procedure for clinicians, and to provide more reliable and stable assessment of the wound status.

### SUMMARY

In view of the above-mentioned problems, it is an object of the present disclosure to provide improvements in the field of sensor systems used in conjunction with wound dressings to provide a reliable, accurate and stable wound status measurements.

In a first aspect, there is provided a sensor strip for use in conjunction with a medical dressing, wherein the sensor strip comprises at least one sensing electrode configured to measure the concentration of dissolved molecules in a wound, and at least a first and a second reference electrode, wherein the at least first and reference second electrodes are individually distinct and differ by at least one property selected from set-up time, long-term-stability, life-time, and stability in response to chemical changes.

The present disclosure is based on the realization that the provision of at least a first and a second reference electrode having distinct properties ensures that the weaknesses of one electrode are compensated by the strengths of another. Reference electrodes play a vital role in stabilizing measurements by the sensing electrode(s) by providing a consistent reference point for the sensing electrode. However, individual reference electrodes may have limitations, such as varying set-up times, or susceptibility to environmental change.

For example, one reference electrode may have a short set-up time, making it ideal for immediate and accurate initial readings, while another reference electrode provides long-term stability, ensuring sustained accuracy over extended periods.

Achieving a balance between e.g. rapid sensor set-up, long-term stability, and accurate, reliable measurements is generally challenging. Often, a sensor that excels in one property, such as having a short set-up time, may perform poorly in another area, such as long-term stability. Conversely, a sensor with excellent long-term stability may require a lengthy set-up time or may be prone to drift over shorter periods. There is typically no single sensor that "has it all".

The sensor strip of the present disclosure secures that the overall accuracy and reliability of the measurements are improved, and that the individual shortcomings of each reference electrode are "outbalanced".

Moreover, the use of at least a first and a second reference electrode allows the system to maintain reliable measurements in the variable environment of the wound. Factors such as changing exudate composition, pH variations, and biofilm formation can affect sensor performance. The complementary properties of the different reference electrodes help to mitigate these challenges, providing more stable and reliable sensor readings.

The sensor strip is to be used in conjunction with a medical dressing. The sensor strip may either be an integrated component of the medical dressing or a separate component that may be detachably attached to the medical dressing.

In exemplary embodiments, the at least one sensing electrode may be configured to measure the concentration of oxygen, glucose, or the concentration of ions selected from hydrogen, sodium, potassium, calcium, magnesium, chloride ions, and/or ammonium ions in the wound.

Each of the above-mentioned dissolved molecules may be indicative of the wound status, e.g. exudate composition or may indicate early signs of infection.

Monitoring the concentration of these dissolved molecules may thus provide critical insight into the biochemical and physiological conditions of the wound. This may trigger the medical personnel to take action and to thereby improve the healing progress.

The oxygen level may indicate tissue oxygenation, which is key for cellular metabolism and energy production. Adequate oxygenation promotes effective wound healing by supporting cell proliferation and collagen synthesis. A low oxygen level (hypoxia) may indicate poor blood supply and may be a sign of wound infection.

Elevated glucose levels in the wound can create an environment that promotes bacterial growth. Monitoring glucose levels can provide insights into the metabolic activity within the wound, helping to assess whether healing is progressing as expected.

The concentration of the ions mentioned hereinbefore may all be indicative of the wound status. For example, an acidic environment (low pH) can promote healing, whereas alkaline conditions (high pH) may indicate infection or a chronic wound status.

Sodium, potassium, calcium, magnesium, chloride, and ammonium ions are essential for maintaining cellular function and osmotic balance within the wound. Imbalances may impair the wound healing process.

Accordingly, maintaining the right balance of ions and oxygen levels creates an optimal environment for wound healing, which may lead to faster recovery and improved outcomes for the patients.

In exemplary embodiments, the sensor strip further comprises at least a third reference electrode, preferably at least a fourth reference electrode.

The incorporation of at least a third, preferably a fourth reference electrode allows for cross-referencing the signals from multiple reference electrodes. Accordingly, the accuracy of measurements may be maintained over extended periods. As one reference electrode begins to drift or degrade, the others can maintain the accuracy and ensure reliable long-term monitoring without the need for frequent replacement or recalibration.

As mentioned hereinbefore, the wound environment may change drastically due to factors like varying exudate composition or the presence of biofilms. The provision of additional reference electrodes may better adapt to these changes such that the sensor strip continues to function accurately under different conditions. Furthermore, if one reference electrode fails or becomes unreliable, the others can continue to provide accurate measurements, reducing the risk of data loss or incorrect readings.

It is also conceivable that the third and/or fourth reference electrodes (or additional reference electrodes) are arranged in the medical dressing, e.g. in a layer of the medical dressing.

In exemplary embodiments, the at least one sensing electrode is a first sensing electrode configured to measure the concentration of hydrogen ions; the sensor strip further comprising a second sensing electrode configured to measure the concentration of oxygen, glucose, or ions selected from sodium, potassium, calcium, magnesium, chloride ions, and/or ammonium ions.

The use of a first sensing electrode measuring hydrogen ions and a second sensing electrode measuring oxygen, glucose, or the ions mentioned hereinbefore enables the simultaneous monitoring of several critical wound status parameters. A more comprehensive and complete assessment of the wound can thus be provided. For example, potential infections can be detected earlier, enabling prompt treatment actions.

In exemplary embodiments, the first reference electrode has a shorter set-up time than the second reference electrode, and wherein the second reference electrode has a longer long-term stability than the first reference electrode.

The first reference electrode, with its shorter set-up time, quickly stabilizes after the medical dressing is applied to the wound. This allows the medical dressing to begin providing accurate measurements almost immediately. This may be important in situations where rapid assessment is necessary, such as detecting early signs of infection or other complications. The second reference electrode, being designed for long-term stability, ensures that the sensor readings remain accurate over an extended period, even when the wound environment changes. This is particularly important for chronic wounds or wounds that require continuous monitoring.

Hence, the quick response of the first electrode provides immediate data, while the second electrode ensures that the accuracy is maintained over time. A robust and reliable sensing system is thus provided.

In exemplary embodiments, the first reference electrode may comprise a first coating; the second reference comprising a second coating, wherein the first coating is different from the second coating such that the first and second reference electrodes are rendered individually distinct.

Accordingly, the at least first and second reference electrodes may be formed from the same electrode material, but the coating characteristics differ between the reference electrodes.

By applying different coatings to the first and second reference electrodes, each electrode can obtain distinct performance characteristics. The first coating may be different from the second coating in thickness or in composition. For example, a thicker coating may slow the electrode's response time but increase its long-term stability, while a thinner coating may provide a faster set-up time but with reduced stability over prolonged use.

In exemplary embodiments, the sensor strip may comprise a polymeric film and an adhesive skin-contact layer; the adhesive skin-contact layer preferably comprising a silicone-based adhesive.

The polymeric film is desirably thin and pliable such that it can stretch and conform to the movement of a wearer. In embodiments where the sensor strip is a separate component which is detachably attached to the medical dressing, the adhesive skin-contact layer secures that the sensor strip remains attached to the skin during use.

A silicone-based adhesive is gentle and non-irritating to the skin. It is a soft and flexible adhesive which conforms well to the body contours without causing trauma upon removal.

In exemplary embodiments, the at least one sensing electrode and at least the first reference electrode each comprises an antibacterial coating.

This prevents the formation of biofilms and inhibits the growth of bacteria on the electrodes. Microbial accumulation may degrade the electrode material and lead to signal drift and a reduced lifespan. The antimicrobial coating secures that the electrodes remain effective throughout their intended use period.

The sensing electrode is preferably arranged in an area where the wound or incision of a patient is located; i.e. in the absorbent pad or below the absorbent pad of a medical dressing. This area is generally more susceptible to microbial contamination. The first reference electrode, which may also be referred to as the "main" reference electrode is typically arranged in close proximity of the sensing electrode. In other words, the first reference electrode is typically also arranged in the area overlying the wound or exudate.

The second reference electrode as well as the at least third and fourth reference electrodes (if present) may also comprise an antimicrobial coating.

According to another aspect, there is provided a medical dressing assembly comprising the sensor strip as described hereinbefore and a medical dressing, wherein the sensor strip is integrated in the medical dressing or is detachably attached to the medical dressing during use.

The detachable configuration may be beneficial to secure a controlled application of the sensor strip (and the medical dressing) onto the wound or incision of a patient. The medical personnel may, in a first step, arrange the sensor strip onto an incision or wound and precisely position the electrodes (particularly the sensing electrode(s) and first reference electrode) with respect to the wound or incision. Subsequently, a separate medical dressing may be arranged on top of the sensor strip and anchored to the strip and to the skin of a patient.

Furthermore, this arrangement allows for the medical dressing to be detached from the sensor strip (and the skin) when saturated i.e. when the dressing has reached its full capacity, and replaced with a new one, while keeping the sensor strip attached to the skin.

The wear time of the sensor strip may thus be significantly prolonged compared to conventional dressings comprising integrated electrodes.

In exemplary embodiments, the medical dressing may comprise a backing layer, an adhesive skin-contact layer, and an absorbent pad arranged between the backing layer and the adhesive skin-contact layer; the absorbent pad being defined by pad edges, wherein the backing layer and the adhesive skin-contact layer are arranged to extend beyond the pad edges to form a border portion surrounding the absorbent pad, and wherein the sensor strip is detachably attached to the adhesive skin-contact layer of the medical dressing during use.

The medical dressing is preferably a so called "border dressing", wherein the centrally arranged pad is configured to handle wound liquid exuding from the wound site, and wherein the border portion (formed by the backing layer and the adhesive skin-contact layer) provides a firm attachment to the skin of a patient.

In exemplary embodiments, the medical dressing and the sensor strip each has a lateral (x) and a longitudinal (y) extension, and wherein the lateral (x) extension of the sensor strip is larger than the lateral (x) extension of the medical dressing.

In embodiments where the sensor strip is detachably attached to the medical dressing, e.g. the adhesive skin-contact layer of the dressing, it may be beneficial that the length (lateral extension) of the sensor strip is larger than that of the medical dressing.

This may be beneficial in order to arrange e.g. the second reference electrode or an additional reference electrode remote from the wound site. It may also be beneficial to simplify the detachment of the medical dressing from the sensor strip (and the skin) when the dressing has reached its full capacity.

In some embodiments, the sensor strip may comprise an electrical connector configured for connection with a separate processing device. The electrical connector may be arranged on a lateral edge of the sensor strip. In other words, the electrical connector may be arranged on the portion of the sensor strip that extends beyond the periphery of an overlying medical dressing. This may be beneficial to enable coupling with a separate processing device in a location remote from the incision or wound and outside the peripheral edges of the medical dressing. This facilitates battery changes, data retrieval, and other maintenance activities without disturbing the wound site or compromising the integrity of the strip/dressing.

In exemplary embodiments, the at least one sensing electrode is arranged in an area of the sensor strip underlying the absorbent pad of the medical dressing.

As mentioned hereinbefore, the sensing electrode is preferably arranged in an area where the wound or incision of a patient is located, i.e. in the area underlying the absorbent pad. In this area, the exudate level is typically high, which allows for more accurate monitoring of dissolved molecules. The first reference electrode is typically also arranged in an area underlying the absorbent pad.

It is also conceivable that the sensor strip is integrated in the absorbent pad of the medical dressing.

According to another aspect, there is provided a wound monitoring system comprising:
- the medical dressing assembly as described hereinbefore, and
- a processing unit configured to analyze the signals from each of the at least sensing electrode and the at least first and second reference electrode to provide real-time monitoring and analysis of a wound condition.

The system allows clinicians to respond quickly to any concerning changes of the wound, e.g. a drop in oxygen level, a significant shift in pH, or any imbalance in the ion concentration, and to take action in a timely manner.

The processing unit is configured to analyze the signals from the electrodes, including i.a. to compare the signals from the multiple reference electrodes and potentially correct for drift or other anomalies.

Furthermore, the processing unit may be configured to issue an alert when specific thresholds are crossed, e.g. when the pH level exceeds a certain limit or when the oxygen level drops below a certain point.

It is also conceivable that the processing unit is integrated with electronic health records, which allows the wound data to be stored, analyzed and shared as part of the patient's overall medical record.

In exemplary embodiments, the wound monitoring system may further comprise a user interface configured to display the analyzed signals of the processing unit and/or information related to the condition of the wound.

The UI allows healthcare providers to easily access, interpret, and respond to real-time data regarding a patient's wound condition. The UI translates the raw data analyzed by the processing unit into easy-to-understand visual formats, such as graphs, charts, and color-coded alerts. This visual representation makes it easier for clinicians to quickly assess the wound's condition and identify any concerning trends or issues.

The wound monitoring system may comprise a wireless interface to send data or alerts to a wireless network, like mobile communications, wlan, or one of many other standards.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a medical dressing assembly comprising a sensor strip according to an exemplary embodiment of the present disclosure.
Figure 2 schematically illustrates a partially split view of a medical dressing for use in conjunction with the sensor strip or forming part of the medical dressing assembly according to an exemplary embodiment of the present disclosure.
Figure 3 schematically illustrates the principles of using a first reference electrode having a short set-up time and a second reference electrode having a longer set-up time, but being long-term stable.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

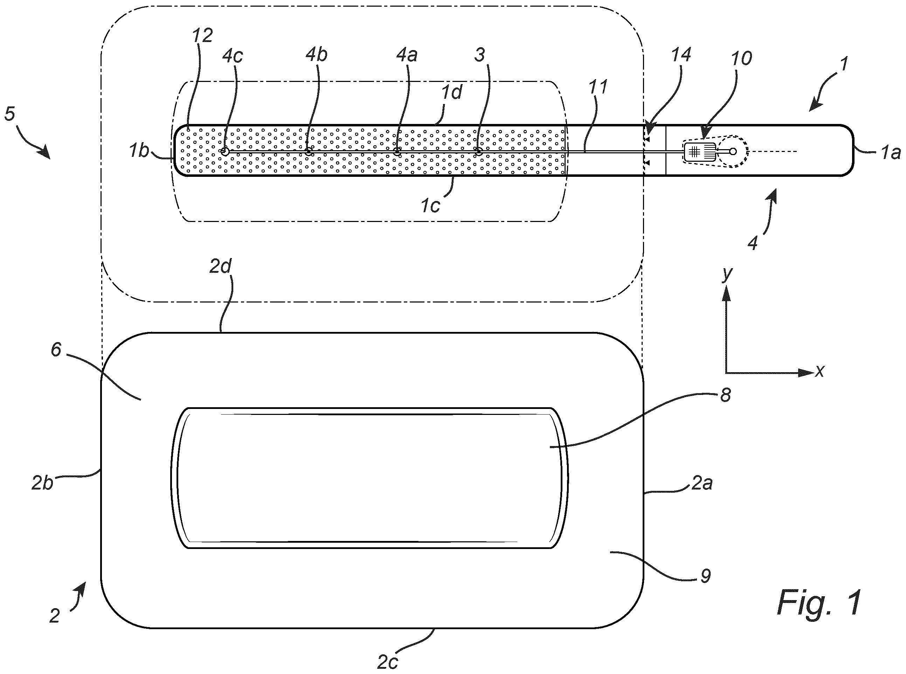

The sensor strip will now be explained with reference to figure. Figure 1 schematically illustrates a sensor strip of the present disclosure for use in conjunction with a medical dressing.

The sensor strip 1 comprises at least one sensing electrode 3 configured to measure the concentration of dissolved molecules in a wound, and at least a first 4a and a second reference electrode 4b, wherein the at least first 4a and second 4b reference electrodes are individually distinct and differing by at least one property selected from set-up time, long-term-stability, life-time, stability in response to chemical changes.

The sensor strip is configured to be used in conjunction with a medical dressing 2. The dotted lines in figure 1 illustrate how the medical dressing 2 may be arranged on the sensor strip 1.

As used herein, the term "sensor strip" means a flexible substrate that houses the at least one sensing electrode and the at least first and second reference electrodes. The electrodes may be connected by an electrical circuit (denoted 11 in figure 1).

The electrodes may be printed onto the sensor strip, e.g. by using a conductive ink. Accordingly, the sensors and the electrical components are firmly attached to the sensor strip and remain functional even when the body moves or when the sensor strip is applied to a contoured or curved body part. It is also conceivable that the electrodes are adhesively attached to the sensor strip.

As used herein, the term "for use in conjunction with a medical dressing" means that the sensor strip may be an integral component of the medical dressing or that the sensor strip is a separate component that is assembled with a medical dressing during use.

In figure 1, the medical dressing is detachably attached to the sensor strip. The present disclosure is by no means limited to the embodiment illustrated in figure 1. It is equally conceivable that the sensor strip is an integral component of the medical dressing. For example, the sensor strip may be fixedly attached to a skin-contact layer of the medical dressing. Alternatively, the sensor strip may be arranged on a layer, e.g. a pad-forming layer of a medical dressing.

As used herein, the term "sensing electrode" means an electrode configured to detect and measure the concentration of dissolved molecules in a wound. The sensing electrode generates an electrical signal that correlates with the concentration of the target analyte, allowing for the real-time monitoring of various wound parameters such as pH, oxygen levels, or ion concentrations.

The sensing electrode may be an ion-selective electrode. The ion-selective electrode is configured to measure specific ions, e.g. hydrogen (H⁺), sodium (Na⁺), potassium (K⁺), calcium (Ca²⁺), magnesium (Mg²⁺), chloride (Cl⁻), and/or ammonium (NH₄⁺) ions. The ion-selective electrode may comprise an ion-selective membrane that responds selectively to the target ion.

Alternatively, the sensing electrode may be an oxygen sensing electrode configured to measure dissolved oxygen levels in the wound. The oxygen sensing electrode may comprise any material (e.g. platinum or gold) which reacts with oxygen to produce a measurable signal.

The sensing electrode may be a potentiometric sensing electrode or an amperometric sensing electrode.

A potentiometric sensing electrode, e.g. an ion-selective sensing electrode, is configured to measure the voltage (electric potential) difference between the at least one sensing electrode and the reference electrodes. The voltage difference correlates with the concentration of a specific ion. The reference electrode provides a stable potential. The difference between these potentials is measured without any significant current flowing through the system.

An amperometric sensing electrode is configured to measure the current that flows in response to a redox (reduction-oxidation) reaction at the surface of the sensing electrode. In the amperometric set-up, the sensing electrode is held at a constant potential relative to the reference electrode, which drives the redox reaction of the target analyte. The current generated by this reaction is measured and is proportional to the concentration of the analyte. An amperometric sensing electrode may be used to measure the concentration of dissolved oxygen and specific molecules, e.g. glucose, that undergo redox reactions. In the amperometric set-up, the incorporation of counter-electrodes is typically required to allow the redox reaction at the sensing electrode to generate a measurable current.

Examples of sensing electrodes include metal or carbon-based electrodes with an ion-selective membrane, polyaniline- or graphene-based electrode, ion-selective field effect transistors, graphene field transistors, or electrodes comprising metal oxides like iridium oxide, ruthenium oxide, titanium oxide.

The term "dissolved molecules" refers to chemical species present in the wound fluid or exudate. The molecules may include gases, such as oxygen or carbon dioxide, as well as small organic or inorganic compounds, such as glucose, lactate, or ions like hydrogen, sodium, potassium, calcium, magnesium, chloride ions, and/or ammonium ions. The concentration of these dissolved molecules provides information about the wound status and healing progress.

As used herein, the term "reference electrode" means an electrode that provides a stable and consistent potential against which the potential of the sensing electrode can be measured. The reference electrode serves as a fixed point of comparison and is configured to maintain its potential regardless of the conditions in the surrounding medium.

The present disclosure is not limited to the use of a specific reference electrode, but any suitable reference electrode known to the skilled person may be used. For example, the reference electrode may be an Ag/AgCl electrode, a Li/LiCl electrode, a Cu/CuSO4 electrode, an Ag/Ag2S electrode or a conductive polymer reference electrode. The reference electrode may also be a metal- or carbon-based electrode covered with a redox couple having a defined potential (e.g. ferrocene or ferrocenium) or a metal oxide or polyaniline-based electrode covered with a pH buffering coating that keeps the H+ concentration constant near the electrode. Preferably, the reference electrode is an Ag/AgCl electrode.

Silver/silver chloride (Ag/AgCl) reference electrodes are reliable and stable over time. Such reference electrodes are non-toxic and compatible with the wound environment such that no adverse side effects are formed in contact with the wound. Furthermore, Ag/Ag Cl electrodes are inexpensive and may be preferred from a cost-efficiency perspective.

That the at least first and second reference electrode are "individually distinct" means that each reference electrode has unique characteristics or properties that differentiate it from the other. These differences can include variations in properties such as set-up time, long-term stability, material composition, coating type, thickness, or response to chemical changes.

The distinct properties allow the electrodes to complement each other, enhancing the overall accuracy and reliability of the sensor system.

The at least first and second reference electrode differ by at least one property selected from set-up time, long-term-stability, life-time, and stability in response to chemical changes.

As used herein, the term "set-up time" means the time required for a reference electrode to stabilize and reach a steady-state potential after being exposed to the wound environment or activated from storage. During this period, the electrode's potential may fluctuate as it adjusts to the surrounding conditions, and accurate measurements can only be obtained once this stabilization is complete.

As used herein, the term "long-term stability" means the ability of a reference electrode to maintain a consistent and accurate potential over an extended period of time, without significant drift or degradation, despite continuous exposure to the wound environment.

As used herein, the term "life-time" refers to the period which a reference electrode remains functional and within its specified performance parameters before it degrades or fails to provide accurate and reliable measurements.

As used herein, the term "stability in response to chemical changes" refers to the ability of a reference electrode to maintain a consistent and accurate potential despite variations in the chemical composition of the wound environment, such as changes in pH, ion concentration, or the presence of interfering substances. This stability ensures that the electrode provides reliable measurements even under fluctuating chemical conditions.

As illustrated in figure 1, the sensor strip may further comprise at least a third reference electrode (4c), preferably at least a fourth reference electrode (not shown).

In figure 1, the electrodes are arranged in a linear sequence. The present disclosure is by no means illustrated to this arrangement, but any arrangement is conceivable.

The at least one sensing electrode 3 is preferably arranged in an area overlying a wound or an incision. When used in conjunction with a medical dressing, the at least one sensing electrode 3 is typically arranged in or below an absorbent pad 8 of the medical dressing 2.

The distance between the sensing electrode 3 and the first reference electrode 4a may be from 0.1 to 100 mm, preferably from 0.1 to 10 mm. The first reference electrode may also be referred to as the "main reference electrode". The potential of the sensing electrode is measured against the main reference electrode.

A distance within this range secures that potential drops or resistive losses in the wound exudate are minimized, which ensures that the potential difference measured by the electrodes is more representative of the actual chemical conditions in the wound. If the distance is too large, small changes in the ion or oxygen concentration may become more difficult to detect.

Typically, the at least first 4a and second 4b reference electrodes differ by a property selected from set-up time, and long-term-stability.

In other words, the first reference electrode 4a may have a shorter set-up time than the second reference electrode 4b, and wherein the second reference electrode 4b has a longer long-term stability than the first reference electrode 4a.

The sensor system thereby allows for a fast response time as well as a prolonged operational period.

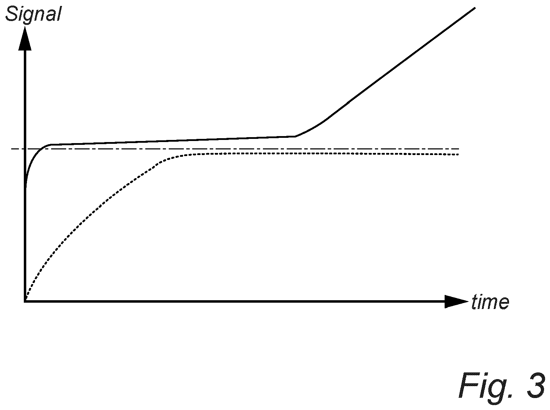

Figure 3 schematically illustrates the principles of using a first reference electrode with a shorter set-up time than a second electrode, wherein the second reference electrode has a longer long-term stability than the first reference electrode.

In figure 3, the central, broken curve illustrates the signal from a "perfect" reference electrode right after it is activated. The solid curve shows the first (main) reference electrode having a short set-up time. As can be seen, the "true value" is reached within a short time (minutes). A reference electrode having a short set-up time typically suffers from a shorter long-term stability, as can be seen in figure 3. The dotted (lower) curve illustrates a second reference electrode having a long long-term stability (days), but the initial signal is not accurate and is also unstable during the first hours. Accordingly, the individual shortcomings of each reference electrode are outbalanced, and the overall accuracy and reliability of the measurements are significantly improved.

The sensor strip may comprise more than one sensing electrode. The sensing electrodes may be configured to measure different dissolved molecules.

For example, the at least one sensing electrode 3 may be a first sensing electrode configured to measure the concentration of hydrogen ions; the sensor strip 1 further comprising a second sensing electrode (not shown) configured to measure the concentration of oxygen, glucose, or ions selected from sodium, potassium, calcium, magnesium, chloride ions, and/or ammonium ions.

In such embodiments, the first sensing electrode may be potentiometric sensing electrode. The second sensing electrode may be a potentiometric sensing electrode (measuring the concentration of ions in the wound) or an amperometric sensing electrode (measuring the concentration of oxygen in the wound).

The first reference electrode 4a may comprise a first coating; the second reference electrode 4b comprising a second coating, wherein the first coating is different from the second coating.

That the first coating is "different" from the second coating means that a property selected from composition or thickness of the coating is different and which renders the at least first and second reference electrodes individually distinct.

By applying different coatings to the first and second reference electrodes, each electrode can obtain distinct performance characteristics. The first coating may be different from the second coating in thickness or composition. For example, a thicker coating may slow the electrode's response time but increase its long-term stability, while a thinner coating may provide a faster set-up time but with reduced stability over prolonged use.

Furthermore, the first coating may differ from the second coating by hydrophilicity, or the concentration of the involved ion.

The first or second coating may comprise a solid state electrolyte (SSE), a salt-containing hydrogel, a conductive polymer, a salt-containing polymer, an ion-selective membrane, or a ceramic material.

Solid state electrolytes (SSEs) provide a stable ion source and maintain a consistent reference potential over time. This is beneficial in the wound environment, where fluctuations in exudate composition could otherwise lead to drift in the reference signal.

Hydrogels containing salts can rapidly hydrate in contact with wound exudate, and quickly establish the necessary ionic environment for the reference electrode to function. This property is useful for achieving a short set-up time, allowing the sensor to provide accurate readings quickly after application. Furthermore, hydrogels help retain moisture around the electrodes which may be important in wound with varying levels of exudate.

In a salt containing polymer, salt particles are integrated in the polymer matrix. This may lead to an enhanced long-term stability since the salt will dissolve slowly, and diffusion of the salt ions from the matrix is limited. Hence, the slow hydration and the slow dissolution of the salt may lead to a prolonged set-up time.

An ion-selective membrane may be used if it is specific to an ion that has a stable concentration in or near the wound. The reference electrode may maintain its stability in the presence of particular ions while being less affected by others.

Ceramic materials may offer high thermal stability and durability and can protect the electrode from degradation over time.

The sensor strip typically comprises a polymeric film. The polymeric film may comprise polyethylene, polyamide, polyester, and/or polyurethane. The thickness of the polymeric film may be in the range of from 10 to 300 µm, preferably from 20 to 70 µm.

In embodiments where the sensor strip is a separate component and is to be assembled with a separate medical dressing during use, the sensor strip may comprise a polymeric film and an adhesive skin-contact layer; the adhesive skin-contact layer preferably comprising a silicone-based adhesive.

Examples of suitable silicone-based adhesives include the two component RTV systems, such as Q72218 (Dow Corning), and SilGel 612 (Wacker Chemie AG), as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4.

The thickness of the adhesive skin-contact layer is typically at least 20 µm. The thickness of the adhesive skin-contact layer may be from 50 to 250 µm.

The polymeric film forms the top side of the sensor strip. The adhesive skin-contact layer forms the bottom (skin-contact) side of the sensor strip.

The sensing electrode 3, the at least first 4a and second 4b reference electrodes, and the electrical circuit 11 may be arranged on the polymeric film of the sensor strip.

As illustrated in figure 1, at least a portion of the sensor strip 1 may comprise a plurality of perforations 12. Preferably, the portion of the sensor strip that is to be arranged below an absorbent pad 8 of a medical dressing 2 comprises a plurality of perforations 12.

Accordingly, wound exudate may pass through the strip and be absorbed by the overlying medical dressing. In this regard, exudate is prevented from accumulating at the wound site, which may cause maceration of the skin and delay the wound healing process. Furthermore, the electrodes are less likely to be obstructed or impaired by exudate buildup.

The perforations 12 extend through the thickness of the sensor strip. In embodiments where the adhesive sensor strip comprises a polymeric film and an adhesive skin-contact layer, the perforations extend through the polymeric film and the adhesive skin-contact layer.

The perforations may be of various shapes and sizes. Preferably, the perforations are arranged in a predetermined, regular pattern. The perforations may have a diameter of from 0.5 mm to 8 mm, e.g. from 1 to 5 mm, preferably from 1 to 2 mm.

The at least one sensing electrode 3 and at least the first reference electrode 4a may each comprises an antimicrobial coating.

This prevents the formation of biofilms and inhibit the growth of bacteria on the electrodes. Microbial accumulation may degrade the electrode material and lead to signal drift and a reduced lifespan. The antimicrobial coating secures that the electrodes remain effective throughout their intended use period.

At least a portion of the sensing electrode and the first reference electrode may be coated with an antimicrobial agent.

Any antimicrobial agent capable of inhibiting the growth and proliferation of microorganisms may be used. For example, the antimicrobial coating may comprise silver, chlorhexidine, chitosan, or quaternary ammonium compounds.

According to another aspect, there is provided a medical dressing assembly 5 comprising the sensor strip 1 as described hereinbefore and a medical dressing 2, wherein the sensor strip 1 is integrated in the medical dressing 2 or is detachably attached to the medical dressing 2 during use.

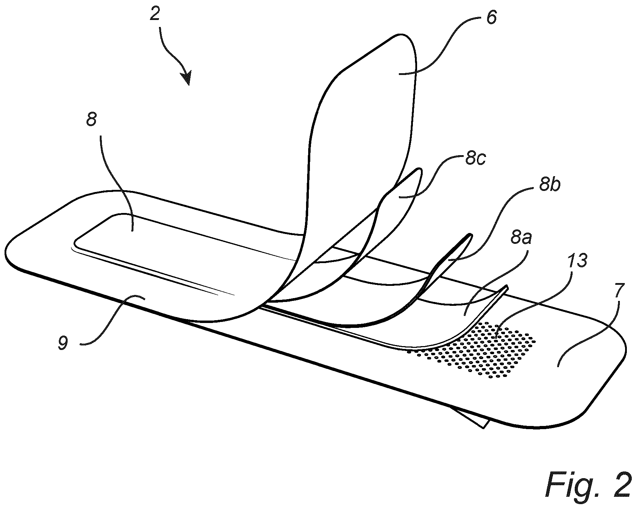

A preferred medical dressing is schematically illustrated in figure 2.

The medical dressing 2 may comprise a backing layer 6 an adhesive skin-contact layer 7, and an absorbent pad 8 arranged between the backing layer 6 and the adhesive skin-contact layer 7; the absorbent pad 8 being defined by pad edges, wherein the backing layer 6 and the adhesive skin-contact layer 7 are arranged to extend beyond the pad edges to form a border portion 9 surrounding the absorbent pad 8.

The "backing layer" is the top layer; i.e. the outermost layer of the medical dressing. The backing layer is a continuous layer and protects the layers of the dressing from entry of potential contaminants. The backing layer typically comprises a polymeric film, e.g. a polyurethane film. The thickness of the backing layer may be in the range of from 15 to 50 µm, preferably from 20 to 40 µm.

The "adhesive skin-contact layer" of the medical dressing is configured to detachably adhere the dressing to a dermal surface. In other words, the adhesive skin-contact layer is configured to contact the skin or the wound or incision. In the context of the present disclosure, the adhesive skin contact layer may also be arranged in contact with the adhesive sensor strip.

The adhesive skin-contact layer 7 may comprise a polymeric film and a silicone-based adhesive coating; the silicone-based adhesive coating being arranged to contact the skin of a wearer during use.

The adhesive skin-contact layer 7 may have the same general construction and contain the same polymeric material and adhesive coating/layer as the sensor strip 1, described hereinbefore.

As illustrated in figure 2, the adhesive skin-contact layer 7 may comprise a plurality of perforations 13. The perforations 13 may be arranged in an area of the adhesive skin-contact layer underlying the absorbent pad 8.

The adhesive skin-contact layer 7 has a first side facing the skin of the patient, and an opposing, second side (illustrated in figure 2).

In embodiments where the sensor strip 1 is integrated in the medical dressing 2, the sensor strip 1 may be arranged on the second side of the adhesive skin-contact layer 7.

Alternatively, the sensor strip 1 may be arranged in the absorbent pad 8.

In the context of the present disclosure, the "absorbent pad" may comprise one or a plurality of pad-forming layers. Typically, the absorbent pad comprises more than one pad-forming layer.

The absorbent pad may e.g. comprise an absorbent polyurethane foam layer.

The absorbent pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the medical dressing.

For example, the absorbent pad 8 illustrated in figure 2 may comprise a first absorbent layer 8a, a liquid distribution layer 8b and a second absorbent layer 8c. Typically, the liquid distribution layer 8b is arranged between the first 8a and the second 8c absorbent layers, wherein the first absorbent layer 8a is the lowermost layer of the absorbent pad.

The first absorbent layer 8a may comprise a foam. Suitable foam materials for use in the first absorbent layer 8a include but are not limited to polyurethane foams.

The second absorbent layer 8c may be a superabsorbent layer. Accordingly, the second absorbent layer may comprise superabsorbent polymers (SAP) or superabsorbent fibers (SAF).

The liquid distribution layer 8b may comprise any material having the ability to distribute the exudate in an efficient manner. For example, the liquid distribution layer 8b may comprise a nonwoven material. A nonwoven imparts an appropriately balanced rigidity to the layer and to the dressing as such. It may also efficiently distribute and spread liquid absorbed by the first absorbent layer 8a such that it can be evaporated through the backing layer 6 over a large surface. For example, the nonwoven may comprise viscose, polyester or blends thereof.

The layers can be joined by adhesion, lamination, using e.g. pressure and heat.

The absorbent pad 8 may comprise additional layers, such as liquid transport layers, various combinations of foam and nonwoven layers laminated together.

In an infected wound, the exudate production is typically large. A medical dressing having the construction as explained hereinabove is suitable for handling large amounts of exudate and for preventing maceration of the skin surrounding the wound.

In the medical dressing assembly 5 illustrated in figure 1, the sensor strip 1 is detachably attached to the adhesive skin-contact layer 7 of the medical dressing. The sensor strip is detachably attached to the skin-facing side of the adhesive skin-contact layer 7.

As used herein, the term "detachably attached" means that the sensor strip is attached to the medical dressing absent a separation force but is capable of being separated from the medical dressing upon the application of a separation force. The detachable attachment is typically formed from the adhesive skin-contact layer of the medical dressing adhering to the sensor strip. That the sensor strip is detachably attached to the medical dressing typically means that the sensor strip is adhesively attached to the medical dressing.

The medical dressing 2 and the sensor strip 1 each has a maximum lateral (x) and a maximum longitudinal (y) extension, and wherein the maximum lateral (x) extension of the sensor strip 1 may be larger than the maximum lateral (x) extension of the medical dressing 2 (see figure 1).

The "maximum lateral (x) extension" may also be referred to as the length of the sensor strip or the medical dressing. The "maximum longitudinal (y) extension" may also be referred to as the width of the sensor strip or the medical dressing. The lateral (x) extension is perpendicular to the longitudinal (y) extension.

The maximum lateral (x) extension of the sensor strip may be from 100 to 600 mm.

The maximum longitudinal (y) extension of the sensor strip may be from 10 to 70 mm, e.g. from 15 to 40 mm.

The maximum lateral (x) extension of the medical dressing may be the same as the maximum lateral (x) extension of the sensor strip or from 10 to 50 mm smaller.

As illustrated in figure 1, the sensor strip is defined by peripheral edges 1a-d. The medical dressing is defined by peripheral edges 2a-d. The peripheral edges of the sensor strip and the medical dressing may be straight or curved. Typically, the sensor strip and the medical dressing each has a substantially rectangular shape. The corners of the medical dressing may be curved. However, the shape of the sensor strip and the medical dressing is not limited to a rectangular shape.

The sensor strip is defined by first 1a and a second 1b lateral edge and a first 1c and a second 1d longitudinal edge. The medical dressing in figure 1 is defined by a first 2a and a second 2b lateral edge and a first 2c and a second 2d longitudinal edge.

A "lateral edge" of the medical dressing, sensor strip or absorbent pad means an edge extending in the longitudinal (y) direction, i.e. parallel to the y axis.

A "longitudinal edge" of the medical dressing, sensor strip, or absorbent pad means an edge extending the lateral (x) direction, i.e. parallel to the x axis.

As illustrated in figure 2, the sensor strip comprises a sensing electrode 3 and a plurality of reference electrodes 4a-c, each of the reference electrodes being individually distinct. The sensor strip 1 may also comprise an electrical connector 10.

As used herein, the term "electrical connector" means a conductive interface component configured to establish an electrical connection between the electrodes and an external processing device, thereby facilitating the transfer of power and data.

The electrical connector 10 may be connected to electrodes by means of an electrical circuit 11. The electrical circuit 11 transmits signals and power between the sensors and an external processing device (which may be connected to the electrical connector 10).

The electrical connector, if present, is preferably arranged in an area of the sensor strip 1 extending beyond a peripheral edge of an overlying medical dressing 2. Accordingly, the electrical connector is arranged remote from the incision or wound. This arrangement facilitates battery changes, data retrieval, and other maintenance activities without disturbing the wound site or compromising the integrity of the strip/dressing. By having the electrical connector positioned away from the wound site and the dressing, there is also less "bulk" and discomfort for the patient.

In some embodiments, the medical dressing which is used in conjunction with the sensor strip is void of electrodes and electronic components. This allows for the major parts of the used materials to be handled as regular medical waste, which significantly reduces the burden on hazardous waste management. Furthermore, the costs associated with several dressing changes can be significantly reduced (since the incorporation of electronic components in a medical dressing is expensive, and also cumbersome).

It is, however, conceivable that the medical dressing 2 further comprises at least one reference electrode.

This may be beneficial to provide a more distributed sensing across the wound area, which may be beneficial in wounds where the exudate level or wound environment varies across different regions. The additional reference electrode, embedded in the medical dressing, may serve as a backup reference point and compensate for the potential shortcomings of the reference electrodes arranged on the sensor strip.

The reference electrode of the medical dressing may be arranged on the second side of the adhesive skin-contact layer 6 or in the absorbent pad 8, e.g. on a pad-forming layer of the absorbent pad 8.

In exemplary embodiments, the sensor strip may further comprise at least one printed marking 14 indicating where a peripheral edge of the medical dressing 2 should be arranged on the sensor strip during use.

The printed marking(s) 14 guide the medical personnel to a correct positioning of the medical dressing. Accordingly, the application procedure for medical personnel is simplified and the likelihood of incorrect placement is eliminated.

According to another aspect, there is provide a wound monitoring system comprising:
- the medical dressing assembly as described hereinbefore, and
- a processing unit configured to analyze the signals from each of the at least sensing electrode and the at least first and second reference electrode to provide real-time monitoring and analysis of a wound condition.

The wound monitoring system may further comprise a user interface configured to display the analyzed signals of the processing unit and/or information related to the condition of the wound.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A sensor strip (1) for use in conjunction with a medical dressing (2), wherein said sensor strip (1) comprises at least one sensing electrode (3) configured to measure the concentration of dissolved molecules in a wound, and at least a first (4a) and a second reference electrode (4b), **characterized in that** said at least first (4a) and second (4b) reference electrodes are individually distinct and differ by at least one property selected from set-up time, long-term-stability, life-time, and stability in response to chemical changes.

2. The sensor strip (1) according to claim 1, wherein said at least one sensing electrode (3) is configured to measure the concentration of oxygen, glucose, or the concentration of ions selected from hydrogen, sodium, potassium, calcium, magnesium, chloride ions, and/or ammonium ions in said wound.

3. The sensor strip (1) according to claim 1 or claim 2, further comprising at least a third reference electrode (4c), preferably at least a fourth reference electrode.

4. The sensor strip (1) according to any one of the preceding claims, wherein said at least one sensing electrode (3) is a first sensing electrode configured to measure the concentration of hydrogen ions; said sensor strip (1) further comprising a second sensing electrode configured to measure the concentration of oxygen, glucose, or ions selected from sodium, potassium, calcium, magnesium, chloride ions, and/or ammonium ions.

5. The sensor strip (1) according to any one of the preceding claims, wherein said first reference electrode (4a) has a shorter set-up time than said second reference electrode (4b), and wherein said second reference electrode (4b) has a longer long-term stability than said first reference electrode (4a).

6. The sensor strip (1) according to any one of the preceding claims, wherein at least one of said at least first (4a) and second (4b) reference electrode is an Ag/AgCl reference electrode.

7. The sensor strip (1) according to any one of the preceding claims, wherein said first reference electrode (4a) comprises a first coating; said second reference electrode (4b) comprising a second coating, wherein said first coating is different from said second coating such that said first (4a) and second (4b) reference electrodes are rendered individually distinct.

8. The sensor strip (1) according to any one of the preceding claims, wherein said sensor strip (1) comprises a polymeric film and an adhesive skin-contact layer; said adhesive skin-contact layer preferably comprising a silicone-based adhesive.

9. A medical dressing assembly (5) comprising the sensor strip (1) according to any one of claims 1-8 and a medical dressing (2), wherein said sensor strip (1) is integrated in said medical dressing (2) or is detachably attached to said medical dressing (2) during use.

10. The medical dressing assembly (5) according to claim 9, wherein said medical dressing (2) comprises a backing layer (6), an adhesive skin-contact layer (7), and an absorbent pad (8) arranged between said backing layer (6) and said adhesive skin-contact layer (7); said absorbent pad (8) being defined by pad edges, wherein said backing layer (6) and said adhesive skin-contact layer (7) are arranged to extend beyond said pad edges to form a border portion (9) surrounding said absorbent pad (8), and wherein said sensor strip (1) is detachably attached to said adhesive skin-contact layer (7) of said medical dressing (2) during use.

11. The medical dressing assembly (5) according to claim 10, wherein said at least one sensing electrode (3) is arranged in an area of said sensor strip (1) underlying said absorbent pad (8) of said medical dressing (2).

12. The medical dressing assembly (5) according to claim 10 or claim 11, wherein said medical dressing (2) and said sensor strip (1) each has a lateral (x) and a longitudinal (y) extension, and wherein the lateral (x) extension of said sensor strip (1) is larger than the lateral (x) extension of said medical dressing (2).

13. The medical dressing assembly (5) according to any one of claims 10-12, wherein said medical dressing (2) further comprises at least one reference electrode.

14. A wound monitoring system comprising:
- the medical dressing assembly according to any one of claims 1-13,
- a processing unit configured to analyze the signals from each of said at least sensing electrode and said at least first and second reference electrode to provide real-time monitoring and analysis of a wound condition.

15. The wound monitoring system according to claim 14, further comprising a user interface configured to display said analyzed signals of said processing unit and/or information related to the condition of the wound.
